# EUROPEAN PATENT SPECIFICATION

(11) **EP 0 463 951 B1**
(45) Date of publication and mention of the grant of the patent: **01.05.1996**
(21) Application number: 91401695.1
(22) Date of filing: 21.06.1991
(51) Int. Cl.: G09G 5/00

(54) **Parallel information display system**
Anzeigesystem für parallele Informationen
Système d'affichage d'informations parallèles

(30) Priority: 25.06.1990 JP 166444/90
(43) Date of publication of application: 02.01.1992
(73) Proprietor: NATIONAL INSTITUTE OF GENETICS, Shizuoka 411 (JP); FUJITSU LIMITED, Kawasaki-shi, Kanagawa 211 (JP)
(72) Inventor: Gojobori, Takashi, Mishima-shi, Shizuoka 411 (JP); Moriyama, Etsuko, Hiratsuka-shi, Kanagawa 254 (JP); Kishino, Atsuko, Toshima-ku, Tokyo 171 (JP); Hirai, Kanako, Kawasaki-shi, Kanagawa 211 (JP); Naito, Kimitoshi, Yokohama-shi, Kanagawa 247 (JP)
(74) Representative: Joly, Jean-Jacques

(56) References cited:
- COMPUTERS AND BIOMEDICAL RESEARCH vol. 23, 1990, SAN DIEGO, CA. USA pages 310 - 331 D. E. FOULSER ET AL. 'Parallel Computation of Multiple Biological Sequence Comparisons'
- DATABASE WPIL Section Ch, Week 18, Derwent Publications Ltd., London, GB; Class D16, Page D, AN 87-129362 & US-A-6 865 403 (US DEPARTEMENT OF HEALTH AND HUMAN) 10 February 1987
- PATENT ABSTRACTS OF JAPAN vol. 013, no. 242 (P-880) 7 June 1989 & JP-A-10 44 853 (FUJITSU LTD.) 17 February 1989

## Description

### BACKGROUND OF THE INVENTION

The present invention generally relates to parallel information display systems, and more particularly to a parallel information display system which enables analysis and editing of information relative to other information which is displayed in parallel therewith. The present invention is particularly suited for application to a genetic information analyzing and editing apparatus.

In biology and biology utilizing fields, there is a demand to realize an editing apparatus which enables simple analysis of a plurality of genetic sequences.

An example of a conventional editing apparatus is disclosed in: Computers and Biomedical Research, vol. 23, 1990, San Diego, CA. USA, pages 310-331, D.E. Foulser et al.: "Parallel Computation of Multiple Biological Sequence Comparisons".

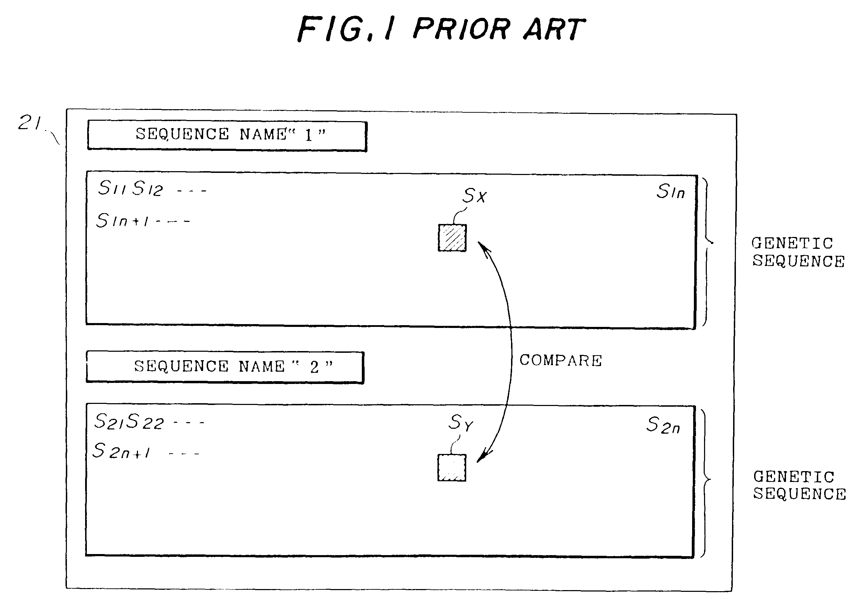

According to another conventional apparatus, when displaying the genetic sequences on a display for analysis and editing the genetic sequences by inserting a gene depending on the analysis, the genetic sequences are displayed as shown in FIG. 1. That is, elements S₁₁, S₁₂, ... of a genetic sequence having a sequence name "1" (for example, human) are displayed within a first block, and elements S₂₁, S₂₂, ... of another genetic sequence having a a sequence name "2" (for example, ape) are displayed within a second block which is independent of the first block. When analyzing the genetic sequences, an element S_{X} within the first block is compared with an element S_{Y} within the second block, for example. An editing is made by inserting a desired element into the genetic sequence displayed within the first or second block so as to improve the degree of similarity of the two genetic sequences, for example.

However, the genetic sequence may have approximately 800 elements, and if the display is only capable of displaying 80 elements per line, the display of one genetic sequence may extend for ten lines. As a result, there is a problem in that it is difficult to compare one element within one of the ten lines of one block with one element within one of the ten lines of another block. The comparison becomes more difficult particularly when the elements to be compared are displayed at non-corresponding positions within the respective blocks. For example, the elements to be compared may be located at a second leftmost position in a fourth line of the first block and at a tenth leftmost position in a third line of the second block, and in this case, the operator must shift his eyes back and forth between the non-corresponding positions within the two blocks when making the analysis.

The editing of the genetic sequence is also difficult because the operator must shift his eyes back and forth between the two blocks in order to judge the similarity of the genetic sequences displayed in the two blocks. The analysis and editing becomes even more difficult when more than two genetic sequences are analyzed and edited simultaneously, since the first block and the last block may be quite separated from each other on the display and the simultaneous display of all of the desired blocks on the display may be impossible.

### SUMMARY OF THE INVENTION

Accordingly, it is a general object of the present invention to provide a useful parallel information display system in which the problems described above are eliminated.

According to the present invention, there is provided a parallel information display system for displaying genetic information, comprising: display means; memory means for storing a plurality of genetic sequences each comprising a sequence name and a number of corresponding elements; and input means for specifying a plurality of genetic sequences to be simultaneously displayed on said display means, in which there is provided: control means, coupled to said display means, said memory means and said input means, for controlling at least a read operation from said memory means in response to said input means so that the sequence names and corresponding elements of the specified sequences are simultaneously displayed on said display means in a plurality of consecutive blocks each of which includes a line corresponding to each specified sequence, the lines within a block being adjacently displayed on said display means, and each displayed line having the same predetermined maximum number of elements with the corresponding elements of each line being aligned element-wise within the block on the display means, each portion of a sequence displayed on a line having the corresponding sequence name also displayed on that line, the simultaneous display in line and element-wise alignment of elements within a block facilitating visual comparison between the portions of said sequences displayed within that block, and in which the control means is operable in an editing mode to add or delete elements to or from a displayed line in one block to change the element-wise alignment of the displayed line of the edited sequence in said one block and the corresponding lines of the edited sequence in all subsequent blocks.

According to the parallel information display system of the present invention, it is possible to display the elements of the sequences to be compared at adjacent lines of the same block, thereby making the comparison easy. Hence, the analysis and editing of the sequences can be made with ease within a relatively short time.

Other objects and further features of the present invention will be apparent from the following detailed description when read in conjunction with the accompanying drawings.

### BRIEF DESCRIPTION OF THE DRAWINGS

Fig. 1 is a diagram for explaining an example of a conventional display arrangement;
Fig. 2 is a system block diagram showing a first embodiment of a parallel information display system according to the present invention;
Fig. 3 is a flow chart for explaining a display process of the third embodiment;
Fig. 4 is a flow chart for explaining an editing process of the first embodiment;
Figs. 5A through 5C are diagrams for explaining an operation of the first embodiment;
FIGS.6A through 6C are diagrams for explaining examples of genetic sequences;
FIG.7 is a system block diagram showing a second embodiment of the parallel information display system according to the present invention;
FIG.8 is a diagram for explaining an operation of the second embodiment; and
FIG.9 is a flow chart for explaining a display process of the second embodiment.

### DESCRIPTION OF THE PREFERRED EMBODIMENTS

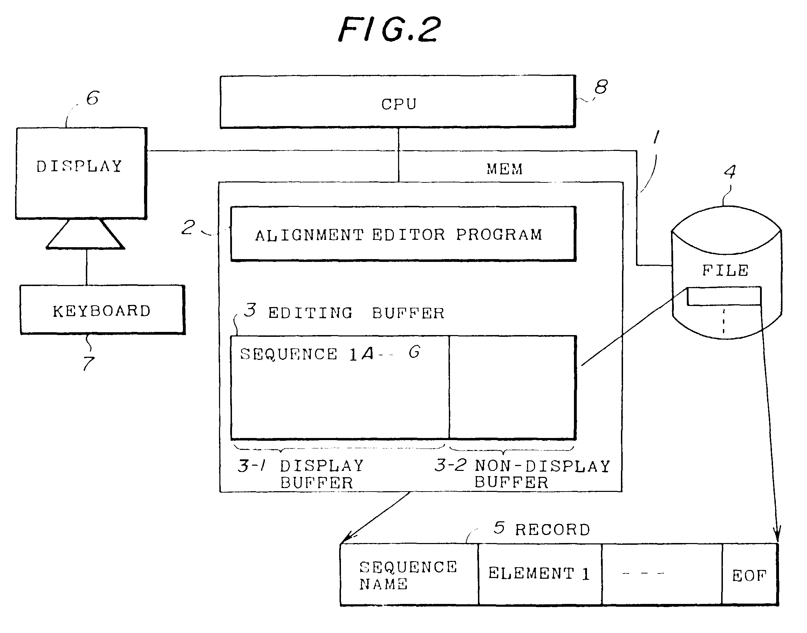

FIG. 2 shows a first embodiment of a parallel information display system according to the present invention. In this embodiment, the present invention is applied to a genetic information analyzing and editing apparatus. The parallel information display system includes a memory 1, a file 4, a display 6, a keyboard 7 and a central processing unit (CPU) 8 which are connected as shown.

The memory 1 includes an alignment editor program 2 and an editing buffer 3. The editing buffer 3 is made up of a display buffer 3-1 and a non-display buffer 3-2. The alignment editor program 2 carries out various editing processes such as storing genetic sequences in the editing buffer 3, while the display buffer 3-1 stores data to be displayed on the display 6. The non-display buffer 3-2 stores overflow data which overflow from the display buffer 3-1. The data stored in the display buffer 3-1 are sequence names of the genetic sequences, elements of the genetic information and the like. For example, the data overflow from the display buffer 3-1 when a character representing the genetic information is inserted at an arbitrary position within the display buffer 3-1. The editing buffer 3 edits and successively displays a plurality of genetic sequences on adjacent lines.

The file 4 stores the genetic information in the form of records 5. As shown in FIG.2, each record 5 includes elements "1", "2", ... and end of file (EOF) in correspondence with each sequence name.

The display 6 displays data such as the data stored in the display buffer 3-1 of the editing buffer 3. The keyboard 7 is used to move a cursor to an arbitrary position of the genetic sequences displayed on the display 6 and to input a character representing the genetic information at the cursor position.

As shown in FIG.2, the alignment editor program 2 successively stores the elements (genes) into the display buffer 3-1 of the editing buffer 3 in units of lines for a plurality of specified sequence names, and one line of elements from each sequence name forms a block. When all of the elements of the sequence name cannot be stored within the block, the remaining elements are stored within a next block and such an operation is repeated. The elements stored in the display buffer 3-1 of the editing buffer 3 are displayed on the display 6, and one or more elements may be inserted into a selected line by moving the cursor to an arbitrary position in the selected line. When the elements stored in the display buffer 3-1 for the selected line overflow from the display buffer 3-1 when this insertion is made, the elements which overflow from the display buffer 3-1 are stored in the non-display buffer 3-2. The elements stored in the non-display buffer 3-2 are stored in the display buffer 3-1 together with the elements of the next line if necessary.

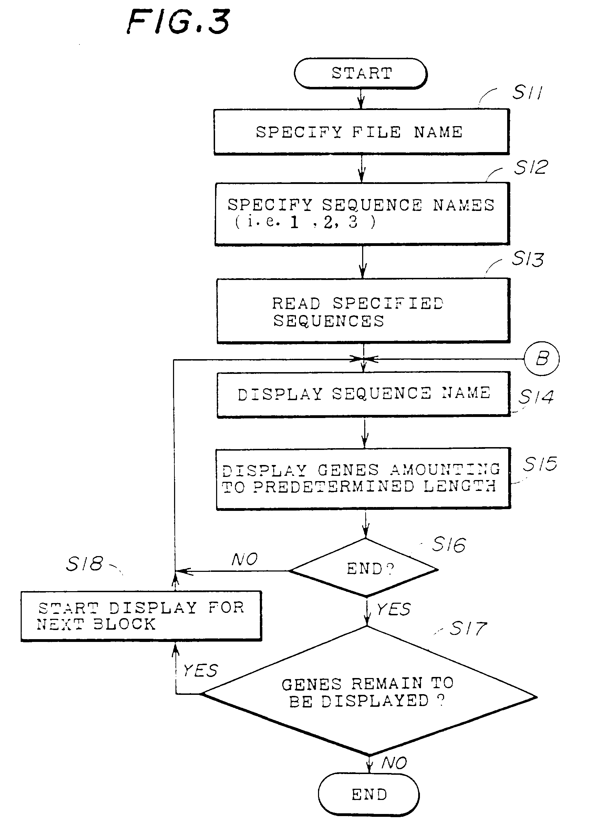

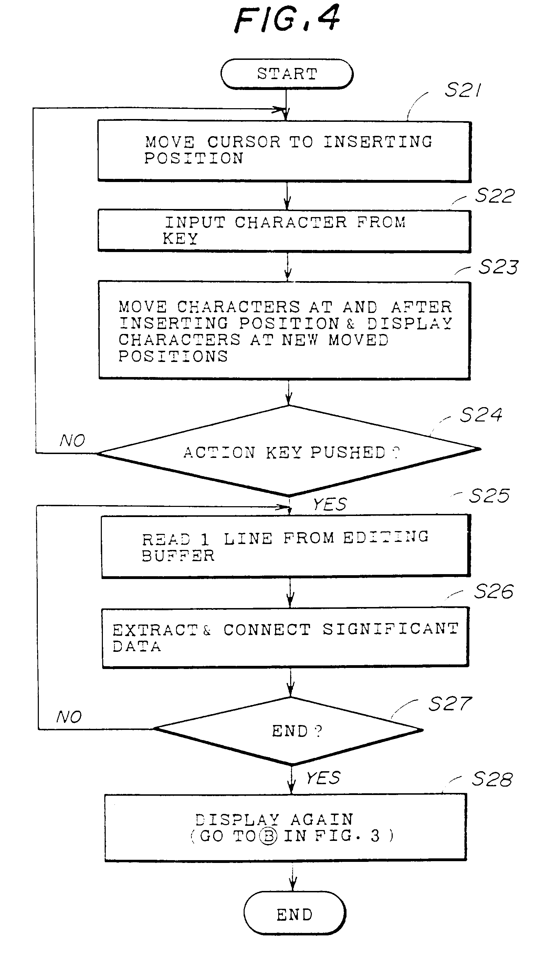

Next, a description will be given of a display process of the first embodiment, by referring to FIG.3. In FIG.3, a step S11 specifies the file name. A step S12 specifies the sequence names. For example, the sequence names "Sequence 1", "Sequence 2" and "Sequence 3" are specified. The file and sequence names are specified from keys of the keyboard 7 by inputting the file and sequence names of a genetic sequence which is to be analyzed and edited.

A step S13 reads the specified genetic sequences. The specified genetic sequences are read by the alignment editor program 2 which reads from the file 4 the records 5 corresponding to the file and sequence names which are specified in the steps S11 and S12, and transfers the records 5 into the memory 1.

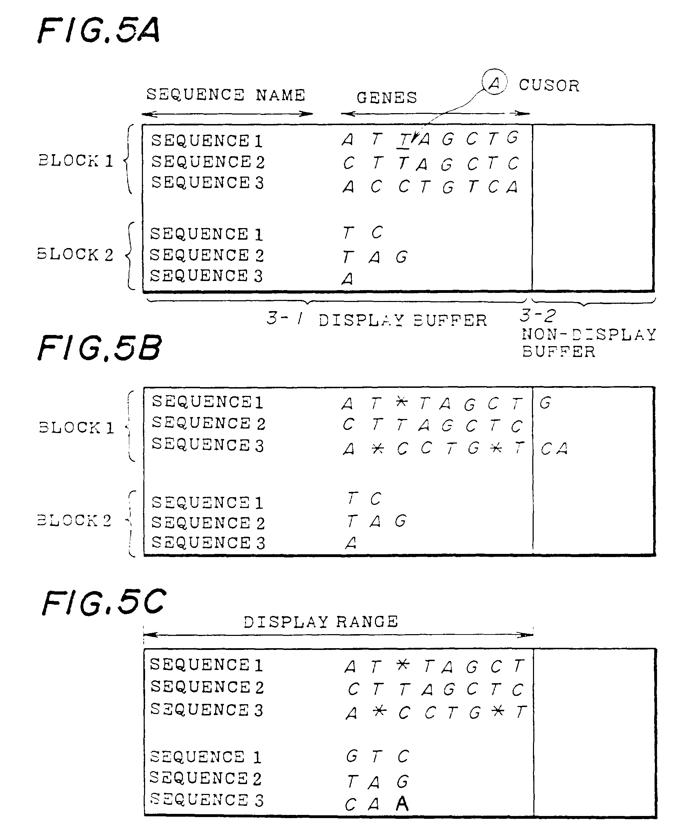

A step S14 displays one of the specified sequence names on the display 6. The sequence name is displayed by extracting the sequence name from the corresponding record 5 which is stored in the memory 1 and storing the sequence name from the start of the display buffer 3-1 of the editing buffer 1. For example, the sequence name "Sequence 1" shown in FIG.5A is displayed on the display 6.

A step S15 displays the genes which amount to to a predetermined length and correspond to the specified sequence name. For example, the genes which amount to one line (predetermined length) and correspond to the sequence name "Sequence 1" are stored in the display buffer 3-1 following the sequence name "Sequence 1". The genes "ATTAGCTG" are stored next to the sequence name "Sequence 1" as shown in FIG.5A.

A step S16 judges whether or not one line of genes is displayed for all of the specified sequence names, that is, whether or not the display of a block 1 shown in FIG.5A is ended. When the judgement result in the step S16 is NO, the process returns to the step S14 and the steps S14 and S15 are repeated. Hence, one line of genes is displayed for each of the sequence names "Sequence 1", "Sequence 2" and "Sequence 3" and the display of the block 1 is made by the repetition of the steps S14 and 15.

On the other hand, when the judgement result in the step S16 is YES, a step S17 judges whether or not one or more genes of the specified sequence names remain to be displayed. When the judgement result in the step S17 is YES, a step S18 starts the display for a next block 2 and the process returns to the step S14. As a result, the next block 2 is stored as shown in FIG.5A and displayed. The process ends when the judgement result in the step S17 becomes NO.

Therefore, the genes (elements) of the genetic sequences having the sequence names specified by the operator are displayed in units of blocks, where each block is made up of one line of each of the specified sequence names. According to this display arrangement, it is easy for the operator to compare and analyze the genes of the specified sequence names on the display.

In FIG.5A and FIGS.5B, 5C, 6A through 6C and 8 which follow, "A" denotes adenine, "G" denotes guanine, "T" denotes thymine and "C" denotes cytosine. In addition, FIGS.5A through 5C respectively show the arrangement of data within the display buffer 3-1 and the non-display buffer 3-2 of the editing buffer 3 in correspondence with the display 6. Hence, the data stored in the display buffer 3-1 are displayed on the display 6 as indicated by "Display Range" in FIG.5C, while the data stored in the non-display buffer 3-2 are not displayed on the display 6. Therefore, the arrangement of the data within the display buffer 3-1 is essentially the same as the display arrangement on the display 6.

Next, a description will be given of an editing process of the first embodiment during an editing mode, by referring to FIG.4. In FIG.4, a step S21 moves the cursor to an arbitrary position where a character or gap is to be inserted by manipulating the keyboard 7. A step S22 inputs the character which is to be inserted from the key of the keyboard 7. For example, the gap may be inserted by inputting a predetermined character or symbol from the keyboard 7.

A step S23 moves the characters which are positioned at and after the inserting position and displays the characters at new moved positions. When one character is inserted, the characters positioned at and after the inserting position are displayed at positions which are respectively shifted by one character from the respective original display positions.

For example, the step S21 moves the cursor to the position of the character "T" in FIG.5A, and the step S22 inputs a character "*" from the keyboard 7 as shown in FIG.5B, As a result, the step S23 moves the characters which are positioned at and after the inserting position by one character and displays the characters at the new moved positions as shown in FIG.5B. In other words, the characters "TAGCTG" positioned at and after the inserting position are displayed at positions shifted by one character to the right from the respective original display positions and the character "*" is displayed at the inserting position.

Thereafter, a step S24 judges whether or not an action key of the keyboard 7 is pushed. This action key specifies a revised display mode in which the edited result is displayed on the display 6. When the judgement result in the step S24 is NO, the process returns to the step S21. On the other hand, when the judgement result in the step S24 is YES, the process advances to a step S25.

The step S25 reads the data amounting to one line from the editing buffer 3. In other words, the sequence name and the corresponding genes in one line are read out from the display buffer 3-1 and the non-display buffer 3-2. A step S26 extracts only the genes which are read in the step S25 and connects the genes. A step S27 judges whether or not all of the genes of the sequence name are read one line at a time and connected for all lines. When the judgement result in the step S27 is NO, the process returns to the step S25 to read the data amounting to another line from the editing buffer 3. The steps S25 and S26 are repeated until the judgement result in the step S27 becomes YES.

When the judgement result in the step S27 is YES, a step S28 displays the genes in units of lines again, so that all of the genes temporarily stored in the non-display buffer 3-2 are now stored in the display buffer 3-1 and displayed on the display 6. The step S28 is carried out by advancing to the step S14 shown in FIG.3. As a result, the data arrangement within the editing buffer 3 and thus the display on the display 6 changes from that shown in FIG.5B to that shown in FIG.5C.

Therefore, by the above described process, the character is inserted in units of lines by moving the cursor to an arbitrary character position of a block and inputting the character from the keyboard 7. The process of displaying the elements of all of the genetic sequences in units of blocks may be carried out again if necessary. Hence, the elements of the genetic sequences to be compared are displayed at adjacent lines so that the analysis (comparison) and the necessary insertion can be made promptly.

As described above, FIGS.5A through 5C show the data stored in the display buffer 3-1 and the non-display buffer 3-2 of the editing buffer 3. Only the data stored in the display buffer 3-1, such as the sequence name and the genes, are displayed on the display 6.

FIG.5A shows an initial state where the genetic sequences "1", "2" and "3" are specified and the data are stored in the display buffer 3-1 by the processes of the steps S11 through S17 shown in FIG.3.

FIG.5B shows a state where one character "*" is inserted in the genetic sequence "1" and the two characters "*" are inserted in the genetic sequence "3". In this case, the rightmost character "G" of the first line of the genetic sequence "1" overflows from the display buffer 3-1 and is stored in the non-display buffer 3-2. Similarly, two rightmost characters "C" and "A" of the first line of the genetic sequence "3" overflow from the display buffer 3-1 and are stored in the non-display buffer 3-2. The characters (data) stored in the non-display buffer 3-2 are not displayed on the display 6.

FIG.5C shows a state after the display process is carried out again in the state shown in FIG.5B. This display is carried out by the processes of the steps S25 through S28 shown in FIG.4 when the action key of the keyboard 7 is pushed in the step S24.

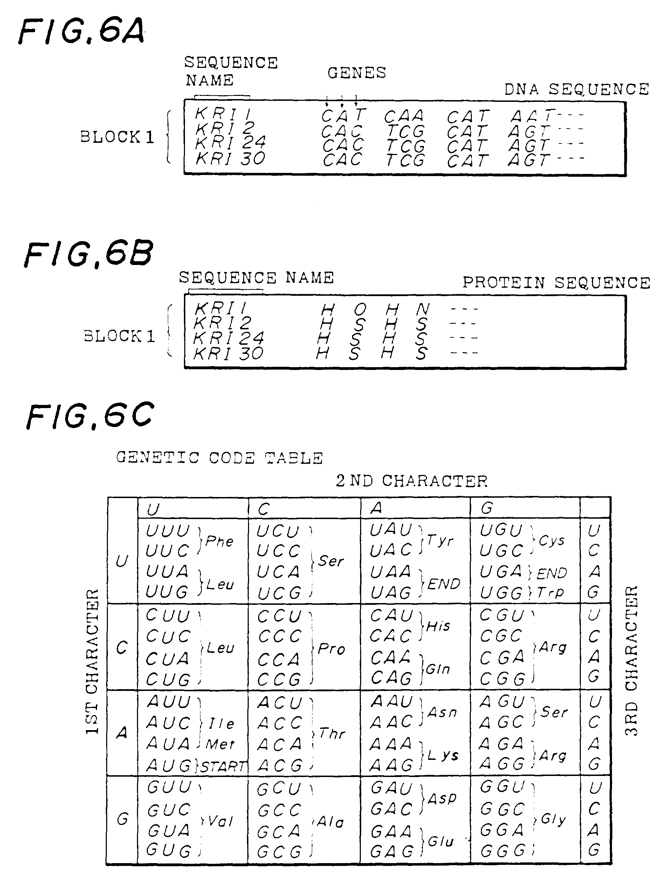

FIG.6A shows DNA sequences as examples of the genetic sequences. The gene in this case is made up of four kinds of bases which are adenine (A), guanine (G), thymine (T) and cytosine (C). When the DNA sequences are displayed in units of blocks as in the first embodiment, the first block displays four sequence names "KRI1", "KRI2", "KRI24" and "KRI30" as shown in FIG.6A, for example.

FIG.6B shows protein sequences as examples of the genetic sequences. Protein is formed when three bases are grouped, and the protein is determined by the combination of the bases. In FIG.6B, "H" denotes histidine, "N" denotes asparagine and "S" denotes serine.

FIG.6C shows a genetic code table which shows the relationship of the grouped bases and the formed protein. In FIG.6C, the following abbreviations are used.
- Ala :: Alanine
- Arg :: Arginine
- Asn :: Asparagine
- Asp :: Aspartic Acid
- Cys :: Cysteine
- Gln :: Glutamine
- Glu :: Glutamic Acid
- Gly :: Glycine
- His :: Histidine
- Ile :: Isoleucine
- Leu :: Leucine
- Lys :: Lysine
- Met :: Methionine
- Phe :: Phenylalanine
- Pro :: Proline
- Ser :: Serine
- Thr :: Threonine
- Trp :: Tryptophan
- Tyr :: Tyrosine
- Val :: Valine

In this embodiment, the inserting operation is described as an example of the editing operation. However, copy, move and delete operations can be carried out similarly as in the case of the inserting operation. In other words, the data which overflows from the display buffer 3-1 is stored in the non-display buffer 3-2. On the other hand, when the number of characters of one line within the block becomes smaller than the maximum number of characters displayed in one line, a unique data such as "0" is stored in the display buffer 3-1 as shown in FIG.6B. When the unique data such as "0" is stored in the display buffer 3-1, only the significant data stored in the display buffer 3-1 are displayed on the display 6 and the unique data "0" is not displayed.

The first embodiment was tested by operating the parallel information display system on the UNIX system. An editing operation was carried out for four Kringle DNA sequences respectively having a length of 234 characters, and genes or gaps amounting to fifteen characters were inserted in a line. It was confirmed that the analysis and the editing operation are facilitated by the display of the sequences in units of blocks each having a line of the sequences to be analyzed.

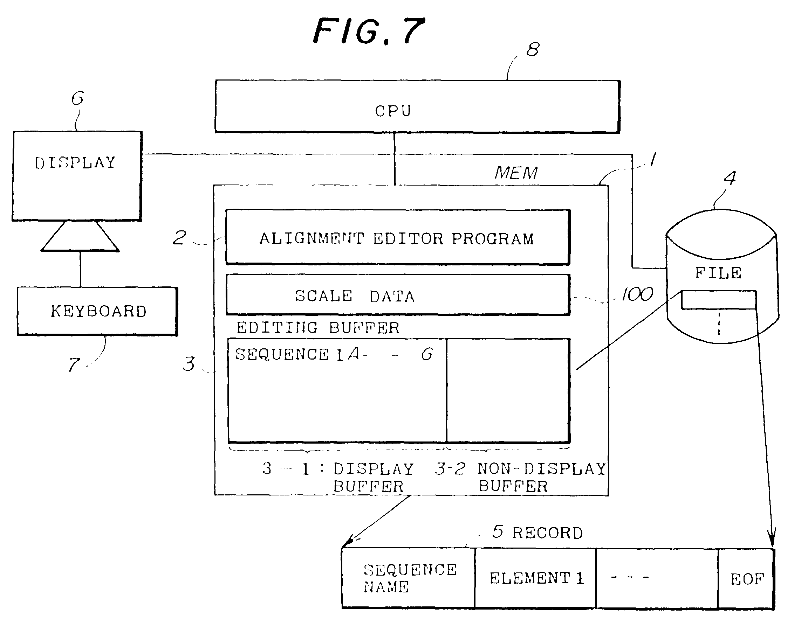

Next, a description will be given of a second embodiment of the parallel information display system according to the present invention. FIG.7 shows the second embodiment. In FIG.7, those parts which are the same as those corresponding parts in FIG.2 are designated by the same reference numerals, and a description thereof will be omitted.

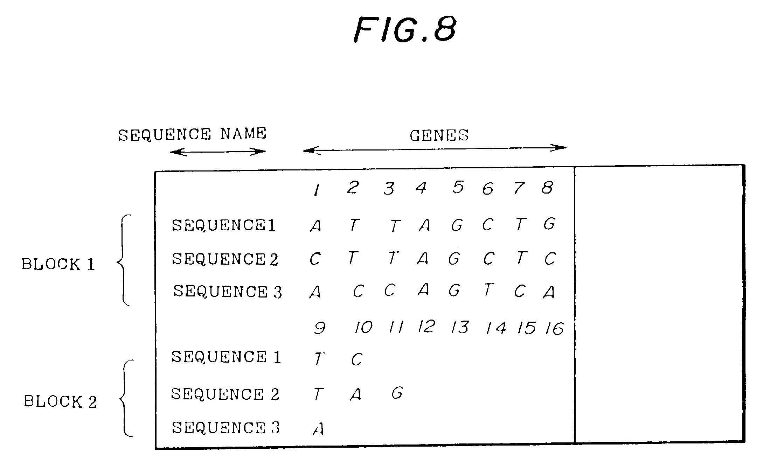

In this second embodiment, the memory 1 shown in FIG.7 includes a scale data 100 for displaying a fixed scale in correspondence with each block so as to facilitate the location of each element of each line within the block. FIG.8 shows an example of the display which is made on the display 6 when the same data shown in FIG.5A are displayed. In FIG.8, the scale indicates the element numbers "1" to "8" in the first block, and indicates the element numbers "9" to "16" in the second block. Although not shown, it is also possible to display the block numbers together with the scale.

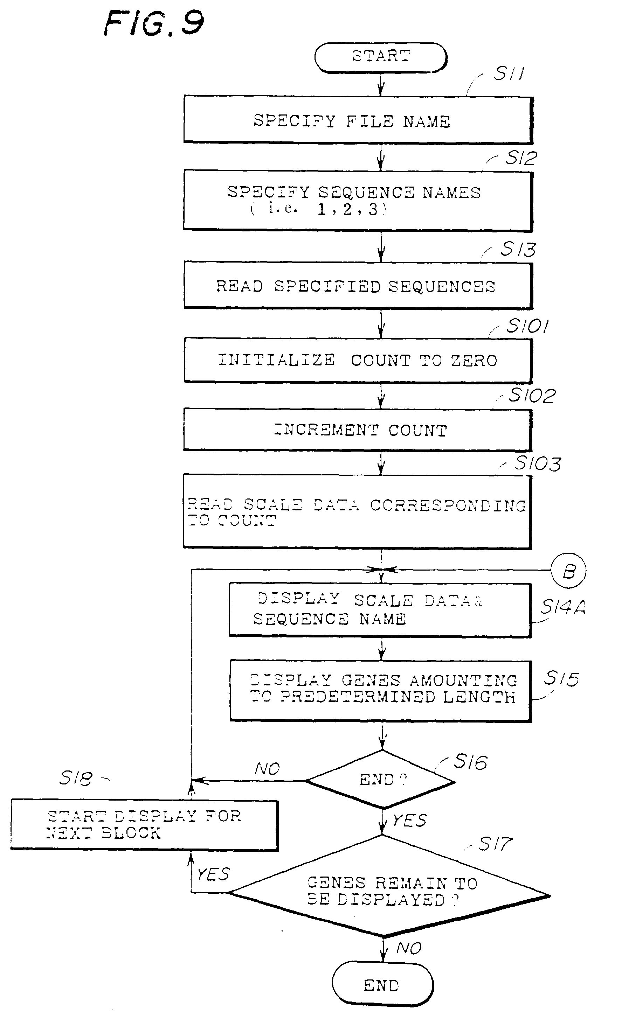

FIG.9 shows a flow chart for explaining the display process of the second embodiment. In FIG.9, those steps which are the same as those corresponding steps in FIG.3 are designated by the same reference numerals, and a description thereof will be omitted. In FIG.9, a step S101 initializes the count to zero. A step S102 increments the count by one, and a step S103 reads the scale data 100 corresponding to the count from the memory 1. The number of characters displayed in one line is known, and is eight in this case. Hence, when the count is "1", the corresponding scale data 100 includes the necessary information to display the scale numbers "1" to "8" as shown in FIG.8. A step S14A displays the scale data 100 read from the memory 1 together with the sequence names in the first block, and the process advances to the step S15. The scale for the second and subsequent blocks are similarly displayed when the sequence names in the subsequence blocks are displayed.

According to this second embodiment, the provision of the scale makes it even easier to locate the elements of the genetic sequences, thereby facilitating the analysis and editing of the genetic sequences.

## Claims

1. A parallel information display system for displaying genetic information, comprising :
display means (6);
memory means (4) for storing a plurality of genetic sequences each comprising a sequence name and a number of corresponding elements; and
input means (7) for specifying a plurality of genetic sequences to be simultaneously displayed on said display means, characterized in that there is provided :
control means (8), coupled to said display means (6), said memory means (4) and said input means (7), for controlling at least a read operation from said memory means (4) in response to said input means (7) so that the sequence names and corresponding elements of the specified sequences are in operation simultaneously displayed on said display means (6) in a plurality of consecutive blocks each of which includes a line corresponding to each specified sequence, the lines within a block being adjacently displayed on said display means (6), and each displayed line having the same predetermined maximum number of elements with the corresponding elements of each line being aligned element-wise within the block on the display means (6), each portion of a sequence displayed on a line having the corresponding sequence name also displayed on that line, the simultaneous display in line and element-wise alignment of elements within a block facilitating visual comparison between the portions of said sequences displayed within that block,
and in that the control means (8) is operable in an editing mode to add or delete elements to or from a displayed line in one block to change the element-wise alignment of the displayed line of the edited sequence in said one block and the corresponding lines of the edited sequence in all subsequent blocks.

2. The parallel information display system as claimed in claim 1, characterized in that there is further provided a memory (1) for storing fixed scale information, and said control means (8) reads the fixed scale information from said memory together with the sequence names and the elements of the specified sequences from said memory means to display on said display means (6) a fixed scale together with each block to indicate positions of each element in the line.

3. The parallel information display system as claimed in claim 1 or 2, characterized in that said input means (7) includes keys which are manipulated during the editing mode in which the elements of an arbitrary block which is displayed on said display means (6) are edited, said editing mode including insertion, deletion, copying and moving of the element.

4. The parallel information display system as claimed in claim 3, characterized in that there are further provided first and second buffer means (3-1, 3-2) for temporarily storing the sequence names and the elements of the specified sequences which are read out from said memory means (4) under a control of said control means (8), said first buffer means (3-1) storing the sequence names and the elements which are to be displayed on said display means (6) in units of blocks, said second buffer means (3-2) storing the elements which overflow from said first buffer means during the editing mode and are not to be displayed on said display means, said control means reading the sequence names and the elements from said first buffer means and displaying the same on said display means.

5. The parallel information display system as claimed in claim 4, characterized in that said control means (8) automatically stores one or more elements of a line which exceed the predetermined number during the editing mode into said second buffer means (3-2) for each line within the arbitrary block.

6. The parallel information display system as claimed in claim 5, characterized in that said input means (7) includes an action key which is manipulated during a revised display mode in which an edited result is displayed on said display means (6), said control means (8) automatically storing the elements stored in said first and second buffer means (3-1, 3-2) into said first buffer means (3-1) in units of blocks when said action key is manipulated.

7. The parallel information display system as claimed in any of claims 1 to 6, characterized in that said control means (8) displays the elements of each block in groups of a predetermined number of elements.

## Patentansprüche

1. Parallel-Informationsanzeigesystem zum Darstellen genetischer Information, umfassend:
- eine Anzeigeeinrichtung (6);
- eine Speichereinrichtung (4) zum Speichern mehrerer genetischer Sequenzen, die jeweils einen Sequenz-Namen und eine Anzahl entsprechender Elemente umfassen; und
- eine Eingabeeinrichtung (7) zum Spezifizieren einer Mehrzahl genetischer Sequenzen, die gleichzeitig auf der Anzeigeeinrichtung darzustellen sind, **dadurch gekennzeichnet,** daß vorgesehen ist:
- eine Steuereinrichtung (8), die an die Anzeigeeinrichtung (6), die Speichereinrichtung (4) und die Eingabeeinrichtung (7) gekoppelt ist, um zumindest einen Lesevorgang aus der Speichereinrichtung (4) ansprechend auf die Eingabeeinrichtung (7) derart zu steuern, daß die Sequenz-Namen und entsprechenden Elemente der spezifizierten Sequenzen im Betrieb gleichzeitig auf der Anzeigeeinrichtung (6) in einer Mehrzahl aufeinanderfolgender Blöcke dargestellt werden, von denen jeder eine Zeile entsprechend jeder spezifizierten Sequenz enthält, die Zeilen innerhalb eines Blocks auf der Anzeigeeinrichtung (6) benachbart dargestellt werden, und jede dargestellte Zeile die gleiche vorbestimmte Maximalzahl von Elementen aufweist, wobei die entsprechenden Elemente jeder Zeile elementweise innerhalb des Blocks auf der Anzeigeeinrichtung (6) ausgerichtet sind, für jeden in einer Zeile dargestellten Abschnitt einer Sequenz ebenfalls in dieser Zeile der entsprechende Sequenz-Name angezeigt wird, wobei die gleichzeitige zeilenweise Anzeige und elementweise Ausrichtung von Elementen innerhalb eines Blocks den visuellen Vergleich zwischen den in dem Block angezeigten Abschnitten der Sequenzen erleichtert,
- und daß die Steuereinrichtung (8) in einer Editierbetriebsart betreibbar ist, um Elemente einer angezeigten Zeile in einem Block hinzuzufügen oder Elemente aus einer angezeigten Zeile in einem Block zu entfernen und so die elementweise Ausrichtung der dargestellten Zeile der editierten Sequenz in dem einen Block und die entsprechenden Zeilen der editierten Sequenz in allen nachfolgenden Blöcken zu ändern.

2. Parallel-Informationsanzeigesystem nach Anspruch 1, **dadurch gekennzeichnet,** daß außerdem ein Speicher (1) zum Speichern fester Skaleninformation vorgesehen ist und die Steuereinrichtung (8) die feste Skaleninformation aus dem Speicher zusammen mit den Sequenz-Namen und den Elementen der spezifizierten Sequenzen aus der Speichereinrichtung ausliest, um auf der Anzeigeeinrichtung (6) eine Festskala zusammen mit jedem Block anzuzeigen und so Positionen jedes Elements in der Zeile anzugeben.

3. Parallel-Informationsanzeigesystem nach Anspruch 1 oder 2, **dadurch gekennzeichnet,** daß die Eingabeeinrichtung (7) Tasten enthält, die während der Editier-Betriebsweise betätigbar sind, in welcher die Elemente eines beliebigen auf der Anzeigeeinrichtung (6) angezeigten Blocks editiert werden, wobei die Editier-Betriebsweise das Einfügen, das Löschen, das Kopieren und das Bewegen des Elements beinhaltet.

4. Parallel-Informationsanzeigesystem nach Anspruch 3, **dadurch gekennzeichnet,** daß zusätzlich eine erste und eine zweite Puffereinrichtung (3-1, 3-2) vorgesehen sind für die vorübergehende Speicherung der Sequenz-Namen und der Elemente der spezifizierten Sequenzen, die auf der Speichereinrichtung (4) unter Steuerung der Steuereinrichtung (8) ausgelesen werden, wobei die erste Puffereinrichtung (3-1) die Sequenz-Namen und die Elemente speichert, die auf der Anzeigeeinrichtung (4) in Block-Einheiten anzuzeigen sind, die zweite Puffereinrichtung (3-2) diejenigen Elemente speichert, die aus der ersten Puffereinrichtung während der Editier-Betriebsweise überlaufen und nicht auf der Anzeigeeinrichtung darzustellen sind, wobei die Steuereinrichtung die Sequenz-Namen und die Elemente aus der ersten Puffereinrichtung ausliest und sie auf der Anzeigeeinrichtung darstellt.

5. Parallel-Informationsanzeigesystem nach Anspruch 4, **dadurch gekennzeichnet,** daß die Steuereinrichtung (8) automatisch ein oder mehrere Elemente einer Zeile, die die vorbestimmte Anzahl während der Editier-Betriebsweise übersteigen, in der zweiten Puffereinrichtung (3-2) für jede Zeile innerhalb des fraglichen Blocks speichert.

6. Parallel-Informationsanzeigesystem nach Anspruch 5, **dadurch gekennzeichnet,** daß die Eingabeeinrichtung (7) eine Aktionstaste beinhaltet, die während einer revidierten Anzeigebetriebsart manipuliert wird, in der ein editiertes Ergebnis auf der Anzeigeeinrichtung (6) dargestellt wird, wobei die Steuereinrichtung (8) automatisch die in der ersten und der zweiten Puffereinrichtung (3-1, 3-2) gespeicherten Elemente in die erste Puffereinrichtung (3-2) in Block-Einheiten speichert, wenn die Aktionstaste betätigt wird.

7. Parallel-Informationsanzeigesystem nach einem der Ansprüche 1 bis 6, **dadurch gekennzeichnet,** daß die Steuereinrichtung (8) die Elemente jedes Blocks in Gruppen einer vorbestimmten Anzahl von Elementen darstellt.

## Revendications

1. Système d'affichage d'informations en parallèle destiné à afficher des informations génétiques, qui comprend :
un moyen d'affichage (6) ;
un moyen de mémorisation (4) destiné à mémoriser une pluralité de séquences génétiques comprenant chacune un nom de séquence et un certain nombres d'éléments correspondants ; et
un moyen d'entrée (7) servant à spécifier une pluralité de séquences génétiques à afficher simultanément sur ledit moyen d'affichage,
caractérisé en ce qu'il est prévu :
un moyen de commande (8), couplé audit moyen d'affichage (6), audit moyen de mémorisation (4) et audit moyen d'entrée (7) et servant à commander au moins une opération de lecture dans ledit moyen de mémorisation (4) en réponse audit moyen d'entrée (7) de façon que les noms de séquence et les éléments correspondants des séquences spécifiées soient, durant le fonctionnement, affichés simultanément sur ledit moyen d'affichage (6) dans une pluralité de blocs consécutifs qui comportent chacun une ligne correspondant à chaque séquence spécifiée, les lignes situées à l'intérieur d'un bloc étant affichées de façon adjacente sur ledit moyen d'affichage (6), et chaque ligne affichée ayant le même nombre maximal prédéterminé d'éléments, les éléments correspondants de chaque ligne étant alignés par élément à l'intérieur du bloc sur le moyen d'affichage (6), chaque partie d'une séquence affichée sur une ligne ayant le nom de séquence correspondant également affiché sur cette ligne, l'affichage simultané en ligne et l'alignement par éléments des éléments à l'intérieur d'un bloc facilitant la comparaison visuelle entre les parties des séquences affichées à l'intérieur de ce bloc,
et en ce que le moyen de commande (8) est en mesure, dans un mode d'édition (mise en forme), d'ajouter ou de supprimer des éléments dans une ligne affichée d'un bloc afin de changer l'alignement par éléments de la ligne affichée de la séquence éditée dans ledit bloc et les lignes correspondantes de la séquence éditée dans tous les blocs suivants.

2. Système d'affichage d'informations en parallèle selon la revendication 1, caractérisé en ce qu'il est en outre prévu une mémoire (1) servant à mémoriser des informations d'échelle fixe, et en ce que ledit moyen de commande (8) lit les informations d'échelle fixe dans ladite mémoire en même temps que les noms de séquence et les éléments des séquences spécifiées dans ledit moyen de mémorisation afin d'afficher sur ledit moyen d'affichage (6) une échelle fixe en même temps que chaque bloc pour indiquer les positions de chaque élément dans la ligne.

3. Système d'affichage d'informations en parallèle selon la revendication 1 ou 2, caractérisé en ce que ledit moyen d'entrée (7) comporte des touches que l'on manipule pendant le mode d'édition dans lequel sont édités les éléments d'un bloc arbitraire qui est affiché sur ledit moyen d'affichage (6), ledit mode d'édition comportant l'insertion, la suppression, la copie et le déplacement de l'élément.

4. Système d'affichage d'informations en parallèle selon la revendication 3, caractérisé en ce qu'il est en outre prévu des premier et deuxième moyens tampons (3-1, 3-2) servant à mémoriser temporairement les noms de séquence et les éléments des séquences spécifiées qui sont lus dans ledit moyen de mémorisation (4) sous commande dudit moyen de commande (8), ledit premier moyen tampon (3-1) mémorisant les noms de séquence et les éléments qui doivent être affichés sur ledit moyen d'affichage (6) en unités de blocs, ledit deuxième moyen tampon (3-2) mémorisant les éléments qui débordent dudit premier moyen tampon pendant le mode d'édition et ne sont pas à être affichés sur ledit moyen d'affichage, ledit moyen de commande lisant les noms de séquence et les éléments dans ledit premier moyen tampon et affichant ceux-ci sur ledit moyen d'affichage.

5. Système d'affichage d'informations en parallèle selon la revendication 4, caractérisé en ce que ledit moyen de commande (8) mémorise automatiquement un ou plusieurs éléments d'une ligne qui sont en excès du nombre prédéterminé pendant le mode d'édition dans ledit deuxième moyen tampon (3-2) pour chaque ligne à l'intérieur du bloc arbitraire.

6. Système d'affichage d'informations en parallèle selon la revendication 5, caractérisé en ce que ledit moyen d'entrée (7) possède une touche d'actionnement que l'on manipule pendant un mode d'affichage révisé dans lequel un résultat édité est affiché sur ledit moyen d'affichage (6), ledit moyen de commande (8) mémorisant automatiquement les éléments mémorisés dans lesdits premier et deuxième moyens tampons (3-1, 3-2) dans ledit premier moyen tampon (3-1) en unités de blocs lorsqu'on manipule ladite touche d'actionnement.

7. Système d'affichage d'informations en parallèle selon l'une quelconque des revendications 1 à 6, caractérisé en ce que ledit moyen de commande (8) affiche les éléments de chaque bloc en groupes d'un nombre prédéterminé d'éléments.
